# EUROPÄISCHE PATENTSCHRIFT

(11) **EP 1 196 097 B1**
(45) Veröffentlichungstag und Bekanntmachung des Hinweises auf die Patenterteilung: **22.10.2003**
(21) Anmeldenummer: 00943955.5
(22) Anmeldetag: 30.06.2000
(51) Int. Cl.: A61B 17/28

(54) **CHIRURGISCHES ROHRSCHAFTINSTRUMENT**
SURGICAL INSTRUMENT WITH A TUBULAR SHAFT
INSTRUMENT CHIRURGICAL A ELEMENT TUBULAIRE

(30) Priorität: 26.07.1999 DE 19935042
(43) Veröffentlichungstag der Anmeldung: 17.04.2002
(73) Patentinhaber: Aesculap AG & Co. KG, 78532 Tuttlingen (DE)
(72) Erfinder: KUPFERSCHMID, Bernhard, D-78576 Emmingen-Liptingen (DE); MORALES, Pedro, D-78532 Tuttlingen (DE); WEISSHAUPT, Dieter, D-78194 Immendingen (DE)
(74) Vertreter: Regelmann, Thomas, Dr.
(86) Internationale Anmeldenummer: EP0006125
(87) Internationale Veröffentlichungsnummer: WO01006937

(56) Entgegenhaltungen:
- DE-U- 29 803 734

## Beschreibung

Die Erfindung betrifft ein chirurgisches Rohrschaftinstrument mit einem Schaft, mit einer in dem Schaft verschieblich gelagerten Arbeitsstange, mit einem Griffteil zur Betätigung der Arbeitsstange und mit Verbindungsmitteln, die das Griffteil und den Schaft lösbar miteinander verbinden, wobei der Schaft in einer inneren Hülse des Griffteils mittels eines in eine Ausnehmung des Schafts eintauchenden, in der inneren Hülse radial verschiebbaren Verriegelungskörpers in axialer ' Richtung festlegbar ist, dessen radiale Auswärtsbewegung durch ein Anschlagelement begrenzt ist, das in eine die radiale Auswärtsbewegung des Verriegelungskörpers freigebende Position verschiebbar ist, und wobei Haltemittel vorgesehen sind, durch die das Anschlagelement in einer Freigabestellung fixierbar ist und die derart durch den Schaft beaufschlagbar sind, daß durch Verschiebung des Schafts in der inneren Hülse diese Fixierung lösbar ist.

Ein solches Rohrschaftinstrument ist beispielsweise in der nicht vorveröffentlichten deutschen Patentanmeldung 198 09 120.6 oder in DE-U-298 03 734 beschrieben.

Bei diesem vorbekannten Rohrschaftinstrument sind auch entsprechende Verbindungsmittel vorgesehen, die die Arbeitsstange lösbar mit dem Griffteil verbinden.

Es ist Aufgabe der Erfindung, den Aufbau eines solchen Instrumentes zu vereinfachen, insbesondere im Hinblick auf eine Verringerung des für die Verbindungsmittel notwendigen Platzbedarfes.

Diese Aufgabe wird bei einem Rohrschaftinstrument der eingangs beschriebenen Art erfindungsgemäß dadurch gelöst, daß die Haltemittel ein Halteelement umfassen, welches außerhalb der inneren Hülse im Griffteil verschieblich geführt ist.

In dem Griffteil muß außerhalb der inneren Hülse ein entsprechender Platz vorgesehen werden, um die Verbindungsmittel anzuordnen. Bei dem in der DE 198 09 120.6 beschriebenen Rohrschaftinstrument ist das Halteelement innerhalb der inneren Hülse verschieblich geführt. Dies erfordert es, daß die innere Hülse einen Innenraum aufweist, welcher einen größeren Durchmesser als der Schaft hat. Durch die erfindungsgemäße Anordnung des Halteelements wird ein Raum, der sowieso bereits vorhanden ist, besser genutzt, und damit läßt sich die innere Hülse kleiner dimensionieren und damit insgesamt das Griffteil kompakter ausbilden und insbesondere kleiner dimensionieren.

Ferner ist es bei der DE 198 09 120.6 vorgesehen, daß die gleichen Verriegelungskörper in der Verbindungsstellung den Schaft mit dem Griffteil verriegeln und mittels des Halteelements die Freigabestellung fixieren. Die Ursache dafür ist, da sowohl der Schaft als auch das Halteelement innerhalb der inneren Hülse geführt sind, daß auch nur eine radiale Inwärtsbewegung eines Verriegelungskörpers eine Verriegelung bewerkstelligen kann. Bei dem erfindungsgemäßen Rohrschaftinstrument läßt sich dagegen die Verriegelung des Schafts und die Verriegelung der Freigabestellung über das Halteelement entkoppeln. Dadurch sind platzsparende Verriegelungsmechanismen erreichbar, die den Aufbau eines erfindungsgemäßen Rohrschaftinstrumentes vereinfachen.

Besonders vorteilhaft ist es, wenn das Halteelement um die innere Hülse verschieblich geführt ist. Die innere Hülse liegt dann innerhalb des Halteelementes. Es läßt sich auf einfache Weise eine Verschiebung des Halteelements gegen das Anschlagelement erreichen, um so für eine Fixierung der Freigabestellung zu sorgen.

Besonders günstig ist es, wenn das Halteelement ein Kopplungselement für den Schaft aufweist, welches in den Innenraum der inneren Hülse weist. Auf diese Weise kann über den Schaft das außerhalb der inneren Hülse liegende Halteelement verschoben werden bzw. das Halteelement kann auf den Schaft einwirken. Damit das Kopplungselement überhaupt auf den Schaft wirken kann, muß die innere Hülse eine Ausnehmung für das Kopplungselement aufweisen.

Besonders günstig ist es dann, wenn der Schaft eine Führungsausnehmung für das Kopplungselement aufweist. Mittels dieser Führungsausnehmung kann über dem Schaft gezielt unter Vermittlung des Kopplungselementes das Halteelement geführt werden, und andererseits kann das Kopplungselement den Schaft entsprechend führen oder festlegen.

Besonders günstig ist es, wenn bei Erreichen der freigebenden Position des Verriegelungskörpers, welcher in der inneren Hülse radial verschieblich ist, das Halteelement eine Vorschubkraft in distaler Richtung auf den Schaft ausübt. Das Kopplungselement kann dann über die Ausnehmungen auf den Schaft einwirken. Das Halteelement funktioniert also als Auswerfer für den Schaft.

Besonders vorteilhaft ist es, wenn durch das Kopplungselement der Schaft bezüglich der inneren Hülse drehfest festlegbar ist. Dadurch wird in der Verbindungsstellung zusätzlich zu der Verriegelung mit den Verriegelungskörpern eine Festlegung des Schaftes in dem Griffteil erreicht. Ein erfindungsgemäßes Rohrschaftinstrument kann dann größere Kräfte und insbesondere größere Drehmomente aufnehmen, ohne daß sich der Schaft löst. Aber die Verriegelungsstellung läßt sich auch einfacher erreichen, da ein Eingriff des Kopplungselements nur noch eine lineare Verschiebung des Schaftes erlaubt. Bei entsprechend angepaßter Ausrichtung zwischen dem Verriegelungskörper und dem Kopplungselement ist dann das Eintauchen des Verriegelungskörpers in die Ausnehmung am Schaft in der Verbindungsstellung gewährleistet.

Günstigerweise ist das Halteelement das Anschlagelement umgebend verschieblich geführt. Dadurch ist einerseits eine Verschiebungsführung für das Halteelement bereitgestellt und andererseits ist der Platzbedarf für diese Verschiebungsführung minimiert.

Günstigerweise sitzt zwischen Halteelement und Anschlagelement eine Feder zur relativen Verschiebung zwischen Halteelement und Anschlagelement, welche in der Verbindungsstellung des Schafts vorgespannt ist. Dadurch läßt sich bei der Lösung der Verbindungsstellung eine automatische Verschiebung des Halteelementes erreichen und so dieses automatisch in eine Stellung überführen, in welcher die Freigabestellung fixiert ist. Auch läßt sich dadurch das Halteelement vorteilhafterweise als Auswerfer für den Schaft bei Lösung der Verbindungsstellung einsetzen.

Besonders vorteilhaft ist es dann, wenn die Federkraft entgegen der Richtung wirkt, in welche das Anschlagelement zur Freigabe der radialen Auswärtsbewegung des Verriegelungskörpers verschiebbar ist. Dadurch läßt sich auf konstruktiv einfache Weise bei der Aufhebung der Verriegelungsstellung eine automatische Fixierung der Freigabestellung erreichen.

Insbesondere um beim Übergang aus der Freigabestellung in die Verriegelungsstellung eine einfache und sichere Rückführung des Halteelementes zu erreichen, ist es vorteilhaft, wenn die zwischen Halteelement und Anschlagelement sitzende Feder so ausgebildet und vorgespannt ist, daß sie in der Verbindungsstellung eine kleinere Federkraft ausübt als eine Rückstellfeder, welche zwischen Anschlagelement und innerer Hülse sitzt und zur Verschiebung des Anschlagelementes aus einer Freigabestellung in die Verbindungsstellung bei gelöster Fixierung des Halteelementes dient. Dadurch kann bei der Rückführung die erstgenannte Feder wieder gespannt werden, um bei Lösen der Verbindungsstellung das Halteelement zu verschieben.

Bei einer konstruktiv besonders einfachen Ausführungsform umfassen die Haltemittel einen in dem Anschlagelement radial verschieblichen Verriegelungskörper, welcher in der Freigabestellung des Anschlagelements in eine Ausnehmung der inneren Hülse eintaucht. Dadurch läßt sich auf einfache Weise das Anschlagelement an der inneren Hülse festlegen, um so die Freigabestellung für den Schaft zu fixieren.

Günstigerweise ist dabei in der Freigabestellung des Anschlagelementes die radiale Auswärtsbewegung des Verriegelungskörpers durch das Halteelement als Anschlag begrenzt. Eine wesentliche Funktion des Halteelementes ist es dann, zur Fixierung der Freigabestellung den Verriegelungskörper zu sperren.

Günstigerweise weist das Halteelement eine Führungsausnehmung für den Verriegelungskörper auf, in der dieser längsverschieblich beim Verschieben des Halteelementes von dessen Stellung bei der Verbindungsstellung in eine Stellung bei der Freigabestellung des Anschlagelementes geführt ist. Diese Führungsausnehmung erlaubt den Übergang zwischen einer definierten Verbindungsstellung und einer definierten Freigabestellung, wobei dann letztere fixierbar ist.

Günstigerweise ist das Anschlagelement als die innere Hülse umgebende Hülse ausgebildet, um so eine sichere Verschiebungsführung zu erreichen und den Raumbedarf zu minimieren.

Weiterhin ist es vorteilhaft, wenn das Halteelement als eine das Anschlagelement umgebende Hülse ausgebildet ist, um ebenfalls eine sichere Verschiebungsführung zu erreichen und den Platzbedarf zu minimieren.

Vorteilhafterweise ist im Griffteil in einem durch das Anschlagelement begrenzten Ringraum eine Führung für das Halteelement gebildet. Dadurch läßt sich wiederum der Platzbedarf minimieren und auf einfache Weise eine Verschiebungsführung ausbilden.

Eine Verbindungsstellung ist auf einfache Weise erreichbar, wenn die Haltemittel ein Halteelement mit einem Kopplungselement aufweisen, durch welches durch Eingriff in ein entsprechendes Gegenelement des Schafts der Schaft bezüglich der inneren Hülse drehfest festlegbar ist.

Auf diese Weise wird zusätzlich zu dem Verriegelungskörper, welcher den Schaft mit der inneren Hülse verriegelt, ein weiteres davon unabhängiges Kopplungselement bereitgestellt, welches den Schaft drehfest festlegt. Das Halteelement ist sowieso bereits vorhanden, um die Freigabestellung zu fixieren. Die zusätzliche Drehsicherung wird daher bei der erfindungsgemäßen Lösung durch minimalen konstruktiven Aufwand erreicht.

Günstigerweise ist dazu das Halteelement außerhalb der inneren Hülse verschieblich geführt, um auf diese Weise den Platzbedarf minimal zu halten.

Weiterhin ist es vorteilhaft, wenn das Gegenelement durch eine Ausnehmung in dem Schaft gebildet ist. Eine solche Ausnehmung läßt sich auf einfache Weise herstellen. Durch Begrenzungswände der Ausnehmung sind Anschlagflächen bereitstellbar, die die Drehsicherung bewirken und andererseits läßt sich aber über derartige Anschlagflächen auch beim Einschieben des Schafts in die innere Hülse über die Kopplungselemente eine Verschiebung des Halteelements zur Fixierung der Freigabestellung erreichen.

Die nachfolgende Beschreibung bevorzugter Ausführungsformen der Erfindung dient im Zusammenhang mit der Zeichnung der näheren Erläuterung. Es zeigen:
- Figur 1:: eine Seitenansicht eines Rohrschaftinstrumentes mit einem koaxial zum Rohrschaft angeordneten Griffteil;
- Figur 2:: eine vergrößerte Längsschnittansicht des Bereiches A in Figur 1 bei verriegeltem Schaft (Verbindungsstellung);
- Figur 3:: eine Ansicht ähnlich Figur 2 bei freigegebenem Schaft (Freigabestellung) und
- Figur 4:: eine Schnittdarstellung längs der Linie 4-4 in Figur 2.

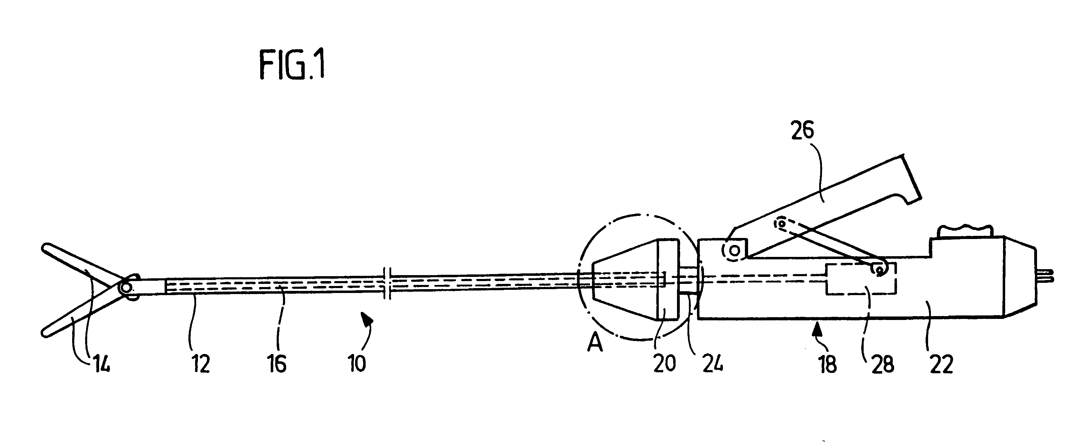

Das in Figur 1 dargestellte und als Ganzes mit 10 bezeichnete Rohrschaftinstrument umfaßt einen rohrförmigen Schaft 12, an dessen distalem Ende zwei bewegbare Werkzeuge 14 gelagert sind, beispielsweise Zangenbacken oder Schneiden eines Schneidwerkzeuges. Im Innern des Schafts 12 ist koaxial zu diesem eine Arbeitsstange 16 und insbesondere eine Schub- und Zugstange längsverschieblich gelagert, die über ein nicht eigens dargestelltes und an sich bekanntes Getriebe die Werkzeuge 14 gegensinnig verschwenkt, wenn die Arbeitsstange 16 gegenüber dem Schaft 12 verschoben wird.

Der Schaft 12 und die Arbeitsstange 16 sind in einem Griffteil 18 gehalten. Das Griffteil 18 weist ein Gehäuseteil 20 auf, in dem der Schaft 12 gehalten ist, und ein Betätigungsteil 22, in dem die Arbeitsstange 16 gehalten ist und über welches diese insbesondere auf Zug und Schub betätigbar ist. Gehäuseteil 20 und Betätigungsteil 22 des Griffteils 18 sind über einen Verbindungsschaft 24, in dem die Arbeitsstange 16 geführt ist, verbunden. Das Betätigungsteil 22 weist einen seitlichen Handgriff 26 auf. Die Arbeitsstange 16 ist mit diesem Handgriff 26 über eine geeignete, in der Zeichnung nur schematisch dargestellte Kopplungseinheit 28 gekoppelt, so daß sie sich über die Betätigung des Handgriffes 26 in Längsrichtung verschieben läßt. Die Kopplungseinheit 28 ist entsprechend so ausgebildet, daß sich die Arbeitsstange 16 bei gelöstem Schaft 12 von der Kopplungseinheit 28 lösen läßt.

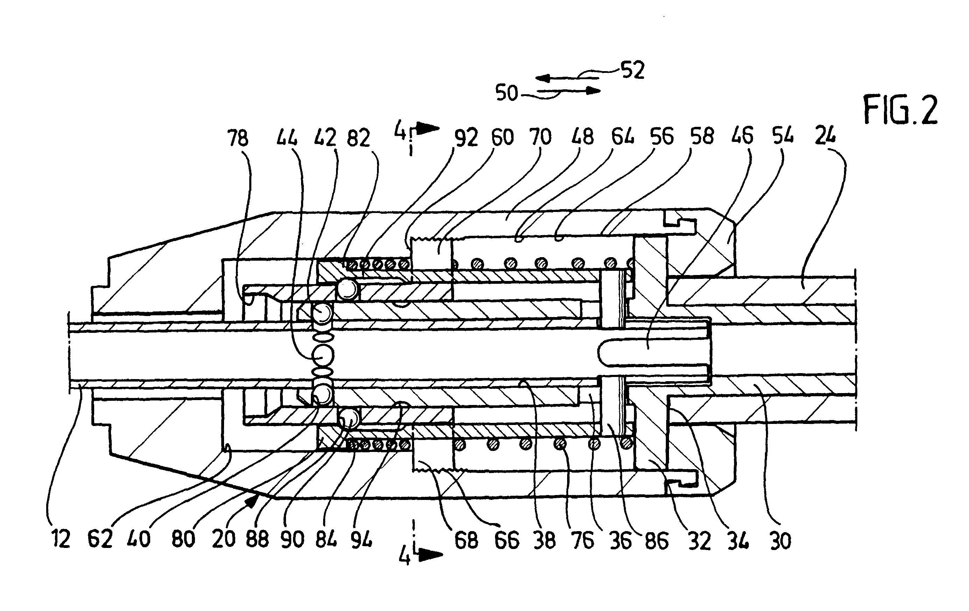

In dem Verbindungsschaft 24 ist koaxial zu diesem eine innere Hülse 30 angeordnet, welche den Schaft 12 aufnimmt und diesen im Gehäuseteil 20 festlegt. Die innere Hülse 30 weist dabei ein radial nach außen weisendes Tellerelement 32 auf, welches an einer vorderen Stirnfläche 34 des Verbindungsschaftes 24 anliegt (Figur 2). Das Tellerelement 32 weist einen in einem kleinen Winkel zur radialen Richtung liegenden Schlitz auf, der zum Einführen einer Druckfeder dient (in der Zeichnung ist dieser Schlitz nicht gezeigt).

In der inneren Hülse 30 sind durchgehende Öffnungen 36 gebildet, durch die auf einen Innenraum 38 der inneren Hülse 30 zugreifbar ist.

Ferner ist in der Nähe ihres vorderen Endes die innere Hülse 30 mit Ausnehmungen 40 versehen, wobei in einer solchen Ausnehmung 40 ein insbesondere kugelförmiger Verriegelungskörper 42 sitzt und in dieser radial verschieblich ist.

Der Schaft 12 weist schalenförmige Ausnehmungen 44 auf, die mit den Verriegelungskörpern 42 korrespondieren und in welche ein solcher Verriegelungskörper 42 zur Festlegung des Schafts 12 in dem Gehäuseteil 20 eintauchen kann. Der Schaft 12 hat an seinem proximalen Ende in axialer Richtung verlaufende nutförmige Ausnehmungen 46, die zum Eingriff eines entsprechenden Kopplungselementes wie unten näher beschrieben dienen, welches über eine Ausnehmung 40 in der inneren Hülse 30 in eine solche Ausnehmung 46 im Schaft 12 eingreifen kann.

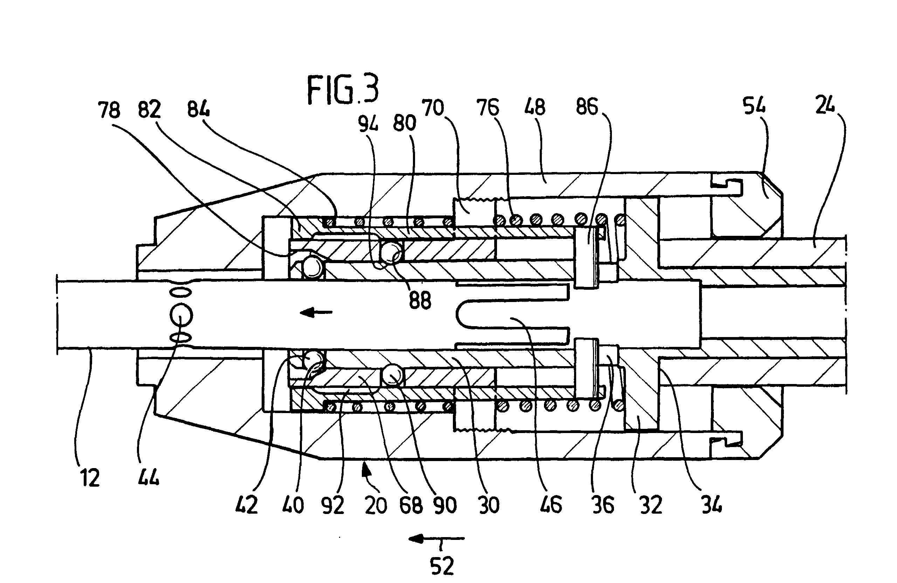

Das Gehäuseteil 20 weist einen Schiebegriff 48 auf, welcher bezüglich des Verbindungsschaftes 24 in axialer Richtung längsverschieblich ist. Bei der in Figur 2 gezeigten Verbindungsstellung, bei der der Schaft 12 in dem Gehäuseteil 20 gehalten ist, ist der Schiebegriff in die proximale Richtung 50 verschiebbar. Bei der in Figur 3 gezeigten Freigabestellung, bei welcher der Schaft 12 vom Gehäuseteil 20 gelöst ist, ist der Schiebegriff 48 in die distale Richtung 52 verschieblich.

An dem hinteren Ende des Schiebegriffes 48 sitzt eine Anschlagmutter 54, welche in der Verbindungsstellung an die dem Verbindungsschaft 24 zugewandte Tellerfläche des Tellerelementes 32 anliegt und so die Verschiebbarkeit des Schiebegriffs 48 in die distale Richtung 52 begrenzt (Figur 2).

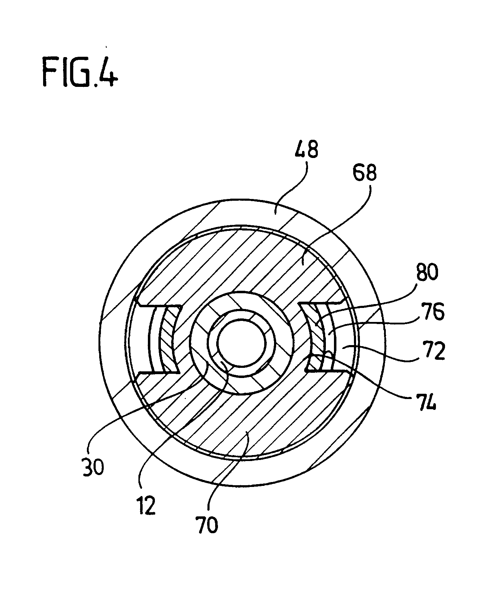

Zwischen dem Schiebegriff 48 und der inneren Hülse 30 ist innerhalb des Gehäuseteils 20 ein ringförmiger Innenraum 56 gebildet. Durch eine Stufe 60 in einer inneren Begrenzung 58 des Schiebegriffs 48 ist der ringförmige Innenraum 56 zweiteilig ausgebildet mit einem vorderen Ringraum 62 und einem hinteren Ringraum 64, welcher wegen der Stufe 60 einen größeren Durchmesser als der vordere Ringraum 62 aufweist. Der Schiebegriff 48 ist zum hinteren Ringraum 64 an der Stufe 60 mit einem Innengewinde 66 versehen. In dieses Innengewinde 66 ist ein hülsenförmig ausgebildetes Anschlagelement 68 verschraubt, so daß dieses mit Verschiebung des Schiebegriffes 48 mitverschoben wird. Das Anschlagelement 68. ist auf der inneren Hülse 30 diese umgebend geführt (Figur 4). An seinem hinteren Ende hat das Anschlagelement 68 eine einstückig daran gebildete Mutter 70, die mit einem Außengewinde zum Eingriff in das Innengewinde 66 des Schiebegriffes 48 versehen ist. Der äußere Durchmesser dieser Mutter 70 entspricht im wesentlichen dem Durchmesser des hinteren Ringraums 64. Die Mutter 70 weist bezogen auf die Längsrichtung des Gehäuseteils 20 durchgehende Ausnehmungen 72 auf, die insbesondere diametral einander gegenüberliegend angeordnet sind (Figur 4). Eine bezüglich der inneren Hülse 30 innere Begrenzungsfläche 74 einer solchen Ausnehmung 72 ist koaxial zum Anschlagelement 68 kreisförmig ausgebildet.

Zwischen der proximalen Stirnfläche der Mutter 70 und der distalen Stirnfläche des Tellerelements 32 sitzt im hinteren Ringraum 64 eine Rückstellfeder 76. Zur Längsverschiebung des Schiebegriffs 48 aus der Verbindungsstellung heraus muß dieser gegen die Rückstellkraft der Rückstellfeder 76, welche in die distale Richtung 52 wirkt, verschoben werden. Die Rückstellkraft der Rückstellfeder 76 bewirkt eine Längsverschiebung des Schiebegriffs 48 aus der Freigabestellung (Figur 3) in die distale Richtung 52 in die Verbindungsstellung, welche in Figur 2 gezeigt ist.

Das Anschlagelement 68 weist an seinem vorderen Ende der inneren Hülse 30 zugewandt eine ringförmige Ausnehmung 78 auf, welche einen sich stetig in radialer Richtung verengenden Bereich hat. Das Anschlagelement 68 dient, wie in Figur 2 gezeigt, zur Begrenzung der radialen Auswärtsbewegung des Verriegelungskörpers 42, wenn das Anschlagelement 68 und damit der Schiebegriff 48 in der Verbindungsstellung sind. Auf diese Weise ist der Schaft 12 durch einen in die schalenförmige Ausnehmung 44 eintauchenden Verriegelungskörper 42 mittels des Anschlagelements 68 an dem Gehäuseteil 20 festlegbar. Die ringförmige Ausnehmung 78 erlaubt, wenn diese, wie in Figur 3 gezeigt, einem Verriegelungskörper 42 gegenüberliegt, eine radiale Auswärtsbewegung des Verriegelungskörpers 42, um so den Schaft 12 freizugeben, d.h. die Kopplung mit der inneren Hülse 30 aufzuheben.

In dem Innenraum 56 ist weiterhin ein hülsenförmig ausgebildetes Halteelement 80 verschieblich angeordnet. Dieses Halteelement 80 ist in dem vorderen Ringraum 62 zwischen dem hülsenförmigen Anschlagelement 68 und der entsprechenden Begrenzung 58 des Schiebegriffs 48 und an der Begrenzungsfläche 74 der Mutter 70 verschieblich geführt. Es erstreckt sich durch die Ausnehmung 72 in der Mutter 70 in den hinteren Ringraum 64. An seinem vorderen (distalen) Ende weist das Halteelemente 80 eine Stufe auf, deren radiale Höhe im wesentlichen dem Abstand zwischen dem Anschlagelement 68 und der Begrenzung 58 des Schiebegriffes 48 im vorderen Ringraum 62 entspricht. Diese Stufe dient als Anlageelement für eine Schraubenfeder 84, welche zwischen dieser Stufe 82 und der Mutter 70 sitzt. In der Verbindungsstellung gemäß Figur 2 ist diese Schraubenfeder 84 vorgespannt, d.h. die Schraubenfeder übt auf das Halteelement 80 über die Stufe 82 eine Federkraft in die distale Richtung 52 aus. Das Halteelement 80 weist als Kopplungselement wirkende Kopplungsstifte 86 auf, die radial nach innen weisend an dem Halteelement 80 sitzen und über die Öffnungen 36 in der inneren Hülse 30 in deren Innenraum 38 ragen. In der Verbindungsstellung gemäß Figur 2 liegen die Kopplungsstifte 86 an der entsprechenden nutförmigen Ausnehmung 46 eines eingeschobenen Schaftes 12 an und begrenzen so die Verschiebbarkeit des Halteelementes 80 in die distale Richtung 52.

In dem Anschlagelement 68 sind in radialer Richtung durchgehende Ausnehmungen 88 angeordnet, die zur Aufnahme von insbesondere kugelförmigen Verriegelungskörpern 90 dienen. Die Verriegelungskörper 90 sind in diesen Ausnehmungen 88 in radialer Richtung verschieblich und weisen einen größeren Durchmesser auf als die entsprechende Wandstärke des Anschlagelementes 68, in welcher die Verriegelungskörper 90 sitzen. Einem solchen Verriegelungskörper 90 zugewandt weist das Halteelement 80 eine Ausnehmung 92 in radialer Richtung auf, welche sich auch in Längsrichtung erstreckt, so daß diese der inneren Hülse 30 zugewandt ringförmig ist. In einer solchen Ausnehmung 92 ist ein Verriegelungskörper 90 führbar. Ferner weist die innere Hülse 30 eine schalenförmige Ausnehmung 94 auf, in die ein solcher Verriegelungskörper 90 eintauchen kann, wenn dieser bis zu dieser Ausnehmung 94 mit dem Anschlagelement 68 in proximaler Richtung in der Ausnehmung 92 geführt verschoben wird. Die Ausnehmung 92 weist dabei eine solche Länge in axialer Richtung auf, daß, wenn ein Verriegelungskörper 90 in die schalenförmige Ausnehmung 94 der inneren Hülse 30 eintaucht, ein ausnehmungsfreier Bereich des Halteelements 80 sich über den Verriegelungskörper 90 schieben kann und dadurch als Anschlagkörper für dessen radiale Auswärtsbewegung wirkt und diese verhindert (Figur 3).

Die Rückstellfeder 76 ist bevorzugterweise so ausgebildet und vorgespannt, daß ihre Federkraft in der Verbindungsstellung größer ist als die der Schraubenfeder 84 zur Verschiebung des Halteelements 80.

Das erfindungsgemäße chirurgische Rohrschaftinstrument funktioniert wie folgt:

Bei der Verbindungsstellung, welche in Figur 2 gezeigt ist, ist der Schaft 12 in dem Gehäuseteil 20 gehalten, d.h. mit dem Griffteil 18 verbunden. Dazu tauchen die Verriegelungskörper 42 in die entsprechenden Ausnehmungen 44 des Schaftes 12 ein und das Anschlagelement 68 liegt so über diesen Verriegelungskörpern 42, daß diese an dem Anschlagelement 68 anliegen, d.h. deren radiale Auswärtsbewegung gesperrt ist. Ferner liegen die Kopplungsstifte 86 des Halteelementes 80 in den Ausnehmungen 46 und sperren so zusätzlich eine Drehbewegung des Schafts 12. Durch Anlage der Kopplungsstifte 86 an dem distalen Ende der Ausnehmungen 46 ist die Verschiebung des Halteelements 80 aufgrund der Kraftwirkung der Schraubenfeder 84 in die distale Richtung 52 gesperrt.

Um den Schaft 12 von dem Gehäuseteil 20 zu lösen, muß ein Bediener den Schiebegriff 48 entgegen der Federkraft der Rückstellfeder 76 in die proximale Richtung 50 verschieben. Dadurch wird die Rückstellfeder 76 zusammengedrückt und übt wie erläutert über die Mutter 70 eine Kraft in die distale Richtung 52 auf das Anschlagelement 68 aus. Durch Verschiebung des Schiebegriffes 48 wird das Anschlagelement 68 mit verschoben und dadurch kann, wenn die Ausnehmung 78 im vorderen Ende des Anschlagelementes 68 die Verriegelungskörper 42 erreicht, ein solcher radial nach außen aus der Ausnehmung 44 ausweichen und dadurch den Schaft 12 freigeben.

Durch Verschiebung des Anschlagelementes 68 werden ebenfalls die darin gelagerten Verriegelungskörper 90 über die Ausnehmung 92 im Halteelement 80 mitgeführt, bis diese in die entsprechenden Ausnehmungen 94 der inneren Hülse 30 eintauchen können. Dadurch weichen die Verriegelungskörper 90 radial nach innen aus und die entsprechenden Abstände beim erfindungsgemäßen chirurgischen Rohrschaftinstrument sind so dimensioniert, daß die freigebende Position für die Verriegelungskörper 42 gerade der Eintauchposition der Verriegelungskörper 90 entspricht. Dadurch wiederum begrenzt der Schaft 12 nicht mehr die Verschiebbarkeit des Halteelements 80 in die distale Richtung 52. Aufgrund der Federkraft der Schraubenfeder 84 wird dann das Halteelement 80 nach vorne verschoben und nimmt dabei durch Anschlag an dem distalen Ende der Ausnehmungen 46 den Schaft 12 nach vorne mit, d.h. das Halteelement 80 wirkt als Auswerfer für den Schaft 12. Durch die Vorwärtsbewegung in die distale Richtung 52 des Halteelementes 80, welche relativ zu dem Anschlagelement 68 erfolgt, schiebt sich das Halteelement 80 über die in die Ausnehmungen 94 eingetauchten Verriegelungskörper 90, so daß deren radiale Auswärtsbewegung mittels des Halteelements 80 gesperrt ist. Dadurch ist aber die Position des Anschlagelements 68 relativ zur inneren Hülse 30 und damit zum Gehäuseteil 20 fixiert und der Schiebegriff 48 ist folglich über die Kopplung mit der Mutter 70 in einer Freigabestellung fixiert, bei der der Schaft 12 freigegeben ist, d.h. nicht mehr am Gehäuseteil 20 gehalten ist (Figur 3). Eine weitere Verschiebung des Halteelements 80 in die distale Richtung 52 ist dadurch gesperrt, daß die Kopplungsstifte 86 an dem vorderen Ende der Öffnungen 36 der inneren Hülse 30 anstehen.

Um ausgehend von dieser Freigabestellung des Gehäuseteils 20 einen Schaft 12 mit dem Griffteil 18 zu verbinden, führt ein Bediener den Schaft 12 in proximaler Richtung 50 in die innere Hülse 30 ein und richtet die Ausnehmungen 46 mit den Kopplungsstiften 86 aus. Durch das vordere Ende dieser Ausnehmungen 46 wird bei Ausübung einer Kraft in die proximale Richtung 50 über die Kopplungsstifte 86 das Halteelement 80 entgegen der Wirkung der Federkraft der Schraubenfeder 84 mitgenommen. Dadurch wird die Ausnehmung 92 über die Verriegelungskörper 90 geführt, so daß diese unter Wirkung der Federkraft der Rückstellfeder 36 radial nach außen ausweichen können und so aus den Ausnehmungen 94 in der inneren Hülse 30 geschoben werden. Dadurch ist die Fixierung des Anschlagelements 68 an der inneren Hülse 30 aufgehoben und die Rückstellfeder 76 schiebt das Anschlagelement 68 in die distale Richtung 52. Auf diese Weise wird mittels des sich verengenden Bereichs der Ausnehmung 78 am vorderen Ende des Anschlagelementes 68 ein Verriegelungskörper 42 radial nach innen gedrückt, so daß er in eine gegenüberliegende Ausnehmung 44 im Schaft 12 eingreifen kann. Das Anschlagelement 68 schiebt sich dann über die innere Hülse 30 derart, daß die radiale Auswärtsbewegung der Verriegelungskörper 42 gesperrt ist. Die Verbindungsstellung ist erreicht, wenn die Verriegelungskörper 42 in die entsprechenden Ausnehmungen 44 in dem Schaft 12 eintauchen und die Anschlagmutter 54 ihre Anschlagstellung erreicht hat. Durch die Ausnehmungen 46, in welche die Kopplungsstifte 86 eingreifen, ist bei der Verschiebung des Anschlagelementes 68 die Verschieblichkeit des Halteelements 80 nach vorne (in die distale Richtung) begrenzt und dieses wird wegen der eine größere Federkraft ausübenden Rückstellfeder 76 in Gegenrichtung zu der Verschiebung des Anschlagelements 68 relativ zu diesem zurückverschoben, so daß die Schraubenfeder 84 gespannt wird und in der Verbindungsstellung eine Kraft ausübt. Dadurch werden mit dem Halteelement 80 auch relativ zu den Öffnungen 36 die Kopplungsstifte 86 zurückverschoben. In der Verbindungsstellung greifen die Kopplungsstifte 86 in die Ausnehmung 46 derart ein, daß sie den Schaft 12 zusätzlich zu den Verriegelungskörpern 42 drehsicher festlegen.

Die Verbindung bzw. Lösung der Arbeitsstange 16 von dem Betätigungsteil 22 erfolgt auf bekannte Weise, beispielsweise derart, daß die Arbeitsstange 16 an ihrem Ende ein kugelförmiges Kopplungselement aufweist und die Kopplungseinheit 28 einen Führungskanal für dieses Kopplungselement aufweist und durch entsprechende Stellung des Handgriffes 26 der Führungskanal so ausrichtbar ist, daß das Kopplungselement von diesem lösbar ist, d.h. entnehmbar ist.

## Patentansprüche

1. Chirurgisches Rohrschaftinstrument mit einem Schaft (12), mit einer in dem Schaft (12) verschieblich gelagerten Arbeitsstange (16), mit einem Griffteil (18) zur Betätigung der Arbeitsstange (16) und mit Verbindungsmitteln, die das Griffteil (18) und den Schaft (12) lösbar miteinander verbinden, wobei der Schaft (12) in einer inneren Hülse (30) des Griffteils (18) mittels eines in eine Ausnehmung (44) des Schafts (12) eintauchenden, in der inneren Hülse (30) radial verschiebbaren Verriegelungskörpers (42) in axialer Richtung festlegbar ist, dessen radiale Auswärtsbewegung durch ein Anschlagelement (68) begrenzt ist, das in eine die radiale Auswärtsbewegung des Verriegelungskörpers (42) freigebende Position verschiebbar ist, **dadurch gekennzeichnet, daß** Haltemittel (80, 90) vorgesehen sind, durch die das Anschlagelement (68) in einer Freigabestellung fixierbar ist und die derart durch den Schaft (12) beaufschlagbar sind, daß durch Verschiebung des Schaftes (12) in der inneren Hülse (30) diese Fixierung lösbar ist, und die Haltemittel ein Halteelement (80) umfassen, welches außerhalb der inneren Hülse (30) im Griffteil (18) verschieblich geführt ist.

2. Instrument nach Anspruch 1, **dadurch gekennzeichnet, daß** das Halteelement (80) um die innere Hülse (30) verschieblich geführt ist.

3. Instrument nach Anspruch 1 oder 2, **dadurch gekennzeichnet, daß** das Halteelement (80) ein Kopplungselement (86) für den Schaft (12) aufweist, welches in den Innenraum (38) der inneren Hülse (30) weist.

4. Instrument nach Anspruch 3, **dadurch gekennzeichnet, daß** die innere Hülse (30) eine Ausnehmung (36) für das Kopplungselement (86) aufweist.

5. Instrument nach Anspruch 3 oder 4, **dadurch gekennzeichnet, daß** der Schaft (12) eine Führungsausnehmung (46) für das Kopplungselement (86) aufweist.

6. Instrument nach einem der Ansprüche 3 bis 5, **dadurch gekennzeichnet, daß** bei Erreichen der freigebenden Position des Verriegelungskörpers (42), welcher in der inneren Hülse (30) radial verschieblich ist, das Halteelement (80) eine Vorschubkraft in distaler Richtung (52) auf den Schaft (12) ausübt.

7. Instrument nach einem der Ansprüche 3 bis 6, **dadurch gekennzeichnet, daß** durch das Kopplungselement (86) der Schaft (12) bezüglich der inneren Hülse (30) drehfest festlegbar ist.

8. Instrument nach einem der vorangehenden Ansprüche, **dadurch gekennzeichnet, daß** das Halteelement (80) das Anschlagelement (68) umgebend verschieblich geführt ist.

9. Instrument nach einem der vorangehenden Ansprüche, **dadurch gekennzeichnet, daß** zwischen Halteelement (80) und Anschlagelement (68) eine Feder (84) zur relativen Verschiebung zwischen Halteelement (80) und Anschlagelement (68) sitzt, welche in der Verbindungsstellung des Schaftes (12) vorgespannt ist.

10. Instrument nach Anspruch 9, **dadurch gekennzeichnet, daß** die Federkraft entgegen der Richtung wirkt, in welche das Anschlagelement (68) zur Freigabe der radialen Auswärtsbewegung des Verriegelungskörpers (42) verschiebbar ist.

11. Instrument nach Anspruch 9 oder 10, **dadurch gekennzeichnet, daß** die zwischen Halteelement (80) und Anschlagelement (68) sitzende Feder (84) so ausgebildet und vorgespannt ist, daß sie in der Verbindungsstellung eine kleinere Federkraft ausübt als eine Rückstellfeder (76), welche zwischen Anschlagelement (68) und innerer Hülse (30) sitzt und zur Verschiebung des Anschlagelements (68) aus einer Freigabestellung in die Verbindungsstellung bei gelöster Fixierung des Halteelements (80) dient.

12. Instrument nach einem der vorangehenden Ansprüche, **dadurch gekennzeichnet, daß** die Haltemittel einen in dem Anschlagelement (68) radial verschieblichen Verriegelungskörper (90) umfassen, welcher in der Freigabestellung des Anschlagelements (68) in eine Ausnehmung (94) der inneren Hülse (30) eintaucht.

13. Instrument nach Anspruch 12, **dadurch gekennzeichnet, daß** in der Freigabestellung des Anschlagelements (68) die radiale Auswärtsbewegung des Verriegelungskörpers (90) durch das Halteelement (80) als Anschlag begrenzt ist.

14. Instrument nach Anspruch 12 oder 13, **dadurch gekennzeichnet, daß** das Halteelement (80) eine Führungsausnehmung (92) für den Verriegelungskörper (90) aufweist, in der dieser längsverschieblich beim Verschieben des Halteelementes (80) von dessen Stellung bei der Verbindungsstellung in eine Stellung bei der Freigabestellung des Anschlagelementes (68) geführt ist.

15. Instrument nach einem der vorangehenden Ansprüche, **dadurch gekennzeichnet, daß** das Anschlagelement (68) als die innere Hülse (30) umgebende Hülse ausgebildet ist.

16. Instrument nach einem der vorangehenden Ansprüche, **dadurch gekennzeichnet, daß** das Halteelement (80) als eine das Anschlagelement (68) umgebende Hülse ausgebildet ist.

17. Instrument nach einem der vorangehenden Ansprüche, **dadurch gekennzeichnet, daß** im Griffteil (18) in einem durch das Anschlagelement (68) begrenzten Ringraum (62) eine Führung für das Halteelement (80) gebildet ist.

18. Chirurgisches Rohrschaftinstrument nach einem der vorangehenden Ansprüche, **dadurch gekennzeichnet, daß** die Haltemittel ein Halteelement (80) mit einem Kopplungselement (86) aufweisen, durch welches durch Eingriff in ein entsprechendes Gegenelement (46) des Schaftes (12) der Schaft (12) bezüglich der inneren Hülse (30) drehfest festlegbar ist.

19. Instrument nach Anspruch 18, **dadurch gekennzeichnet, daß** das Gegenelement durch eine Ausnehmung (46) in dem Schaft (12) gebildet ist.

20. Instrument nach Anspruch 18 oder 19, **dadurch gekennzeichnet, daß** das Halteelement (80) außerhalb der inneren Hülse (30) verschieblich geführt ist.

## Claims

1. Surgical, tubular-shafted instrument comprising a shaft (12), an operating rod (16) mounted for displacement in the shaft (12), a handle part (18) for actuating the operating rod (16) and connecting means connecting the handle part (18) and the shaft (12) detachably to one another, the shaft (12) being securable in axial direction in an inner sleeve (30) of the handle part (18) by means of a locking member (42) displaceable radially in the inner sleeve (30) and dipping into a recess (44) of the shaft (12), the radial outward movement of the locking member being limited by a stop element (68) displaceable into a position releasing the radial outward movement of the locking member (42), **characterized in that** holding means (80, 90) are provided for fixing the stop element (68) in a release position, the holding means being able to be acted upon by the shaft (12) in such a way that this fixing is releasable by displacement of the shaft (12) in the inner sleeve (30), and the holding means comprising a holding element (80) guided for displacement in the handle part (18) outside the inner sleeve (30).

2. Instrument in accordance with claim 1, **characterized in that** the holding element (80) is guided for displacement about the inner sleeve (30).

3. Instrument in accordance with claim 1 or 2, **characterized in that** the holding element (80) has a coupling element (86) for the shaft (12) pointing into the interior (38) of the inner sleeve (30).

4. Instrument in accordance with claim 3, **characterized in that** the inner sleeve (30) has a recess (36) for the coupling element (86).

5. Instrument in accordance with claim 3 or 4, **characterized in that** the shaft (12) has a guiding recess (46) for the coupling element (86).

6. Instrument in accordance with any one of claims 3 to 5, **characterized in that** the holding element (80) exerts an advancing force on the shaft (12) in distal direction (52) when the release position of the locking member (42), which is radially displaceable in the inner sleeve (30), is reached.

7. Instrument in accordance with any one of claims 3 to 6, **characterized in that** the shaft (12) is securable by the coupling element (86) so as to be non-rotatable with respect to the inner sleeve (30).

8. Instrument in accordance with any one of the preceding claims, **characterized in that** the holding element (80) is guided for displacement surrounding the stop element (68).

9. Instrument in accordance with any one of the preceding claims, **characterized in that** a spring (84) for the relative displacement between holding element (80) and stop element (68) is seated between holding element (80) and stop element (68) and is biased in the connection position of the shaft (12).

10. Instrument in accordance with claim 9, **characterized in that** the spring force acts opposite to the direction in which the stop element (68) is displaceable for the release of the radial outward movement of the locking member (42).

11. Instrument in accordance with claim 9 or 10, **characterized in that** the spring (84) seated between holding element (80) and stop element (68) is designed and biased so as to exert a smaller spring force in the connection position than a return spring (76) seated between stop element (68) and inner sleeve (30) and serving to displace the stop element (68) out of a release position into the connection position when the fixing of the holding element (80) is released.

12. Instrument in accordance with any one of the preceding claims, **characterized in that** the holding means comprise a locking member (90) displaceable radially in the stop element (68) and dipping into a recess (94) of the inner sleeve (30) in the release position of the stop element (68).

13. Instrument in accordance with claim 12, **characterized in that** the radial outward movement of the locking member (90) is limited in the release position of the stop element (68) by the holding element (80) as stop.

14. Instrument in accordance with claim 12 or 13, **characterized in that** the holding element (80) has a guiding recess (92) for the locking member (90), in which the locking member is guided for longitudinal displacement during the displacement of the holding element (80) from its position in the connection position into a position in the release position of the stop element (68).

15. Instrument in accordance with any one of the preceding claims, **characterized in that** the stop element (68) is designed as a sleeve surrounding the inner sleeve (30).

16. Instrument in accordance with any one of the preceding claims, **characterized in that** the holding element (80) is designed as a sleeve surrounding the stop element (68).

17. Instrument in accordance with any one of the preceding claims, **characterized in that** a guide for the holding element (80) is formed in the handle part (18) in an annular space (62) limited by the stop element (68).

18. Surgical, tubular-shafted instrument in accordance with any one of the preceding claims, **characterized in that** the holding means have a holding element (80) with a coupling element (86) for securing the shaft (12) non-rotatably with respect to the inner sleeve (30) by engagement in a corresponding counterelement (46) of the shaft (12).

19. Instrument in accordance with claim 18, **characterized in that** the counterelement is formed by a recess (46) in the shaft (12).

20. Instrument in accordance with claim 18 or 19, **characterized in that** the holding element (80) is guided for displacement outside the inner sleeve (30).

## Revendications

1. Instrument chirurgical à fût tubulaire comportant un fût (12), une tige de travail (16) montée à déplacement dans le fût (12), une partie de poignée (18) pour actionner la tige de travail (16) et des moyens de liaison qui relient la partie de poignée (18) et le fût (12) l'une à l'autre de manière détachable, le fût (12) pouvant être immobilisé en direction axiale dans une douille (30) intérieure de la partie de poignée (18) au moyen d'un corps de verrouillage (42) plongeant dans un évidement (44) du fût (12) et déplaçable radialement dans la douille (30) intérieure, dont le mouvement radial vers l'extérieur est limité par un élément de butée (68) qui est déplaçable jusque dans une position libérant le mouvement radial du corps de verrouillage vers l'extérieur (42), **caractérisé en ce qu'**il est prévu des moyens de retenue (80, 90) qui permettent de fixer l'élément de butée (68) dans une position de libération et qui peuvent être sollicités par le fût (12) de telle manière qu'en déplaçant le fût (12) dans la douille (30) intérieure, cette fixation peut être détachée et **en ce que** les moyens de retenue comprennent un élément de retenue (80) qui est guidé de manière déplaçable dans la partie de poignée (18) à l'extérieur de la douille (30) intérieure.

2. Instrument selon la revendication 1, **caractérisé en ce que** l'élément de retenue (80) est guidé de manière déplaçable autour de la douille (30) intérieure.

3. Instrument selon l'une ou l'autre des revendications 1 et 2, **caractérisé en ce que** l'élément de retenue (80) présente un élément d'accouplement (68) pour le fût (12), qui est orienté vers l'espace intérieur (38) de la douille (30) intérieure.

4. Instrument selon la revendication 3, **caractérisé en ce que** la douille (30) intérieure présente un évidement (36) pour l'élément d'accouplement (86).

5. Instrument selon l'une ou l'autre des revendications 3 et 4, **caractérisé en ce que** le fût (12) présente un évidement de guidage (46) pour l'élément d'accouplement (86).

6. Instrument selon l'une des revendications 3 à 5, **caractérisé en ce que** lorsque l'élément de retenue (80) a atteint la position de libération du corps de verrouillage (42) qui est déplaçable radialement dans la douille (30) intérieure, il exerce une force d'avancement en direction distale (52) sur le fût (12).

7. Instrument selon l'une des revendications 3 à 6, **caractérisé en ce que** par l'élément d'accouplement (86), le fût (12) peut être immobilisé de manière solidaire en rotation par rapport à la douille (30) intérieure.

8. Instrument selon l'une des revendications précédentes, **caractérisé en ce que** l'élément de retenue (80) est guidé en déplacement en entourant l'élément de butée (68).

9. Instrument selon l'une des revendications précédentes, **caractérisé en ce qu'**entre l'élément de retenue (80) et l'élément de butée (68) se trouve un ressort (84) pour un déplacement relatif entre l'élément de retenue (80) et l'élément de butée (68), lequel est précontraint dans la position de liaison du fût (12).

10. Instrument selon la revendication 9, **caractérisé en ce que** la force de ressort agit à l'encontre de la direction dans laquelle l'élément de butée (68) peut être déplacé pour libérer le mouvement radial du corps de verrouillage (42) vers l'extérieur.

11. Instrument selon l'une ou l'autre des revendications 9 et 10, **caractérisé en ce que** le ressort (84) situé entre l'élément de retenue (80) et l'élément de butée (68) est réalisé et précontraint de manière telle que dans la position de liaison, il exerce une force de ressort plus petite qu'un ressort de rappel (76) qui est situé entre l'élément de butée (68) et la douille (30) intérieure et qui sert à déplacer l'élément de butée (68) depuis une position de libération jusque dans la position de liaison lorsque la fixation de l'élément de retenue (80) est détachée.

12. Instrument selon l'une des revendications précédentes, **caractérisé en ce que** les moyens de retenue comprennent un corps de verrouillage (90) à déplacement radial dans l'élément de butée (68), lequel plonge dans un évidement (94) de la douille (30) intérieure lorsque l'élément de butée (68) est en position de libération.

13. Instrument selon la revendication 12, **caractérisé en ce que** dans la position de libération de l'élément de butée (68), le mouvement radial du corps de verrouillage (90) vers l'extérieur est limité par l'élément de retenue (80) servant de butée.

14. Instrument selon l'une ou l'autre des revendications 12 et 13, **caractérisé en ce que** l'élément de retenue (80) présente un évidement de guidage (92) pour le corps de verrouillage (90), dans lequel ce dernier est guidé en translation lors du déplacement de l'élément de retenue (80) depuis sa position lors de la position de liaison jusque dans une position lors de la position de libération de l'élément de butée (68).

15. Instrument selon l'une des revendications précédentes, **caractérisé en ce que** l'élément de butée (68) est réalisé sous forme de douille entourant la douille (30) intérieure.

16. Instrument selon l'une des revendications précédentes, **caractérisé en ce que** l'élément de retenue (80) est réalisé sous forme de douille entourant l'élément de butée (68).

17. Instrument selon l'une des revendications précédentes, **caractérisé en ce que** dans la partie de poignée (18) est formé un guidage pour l'élément de retenue (80) dans un espace annulaire (62) limité par l'élément de butée (68).

18. Instrument chirurgical à fût tubulaire selon l'une des revendications précédentes, **caractérisé en ce que** les moyens de retenue présentent un élément de retenue (80) avec un élément d'accouplement (86) qui permet, par engagement dans un élément antagoniste (46) correspondant du fût (12), d'immobiliser de manière solidaire en rotation le fût (12) par rapport à la douille (30) intérieure.

19. Instrument selon la revendication 18, **caractérisé en ce que** l'élément antagoniste est formé par un évidement (46) dans le fût (12).

20. Instrument selon l'une ou l'autre des revendications 18 et 19, **caractérisé en ce que** l'élément de retenue (80) est guidé en déplacement à l'extérieur de la douille (30) intérieure.
